# EUROPEAN PATENT APPLICATION

(11) **EP 2 460 415 A1**
(43) Date of publication of application: **06.06.2012**
(21) Application number: 10804338.1
(22) Date of filing: 23.07.2010
(51) Int. Cl.: A23K 1/16, A61K 9/50, A61K 31/05, A61K 31/60, A61K 36/00, A61K 36/18, A61K 47/44, A61P 1/00, A61P 31/04, A61P 33/02

(54) **COATED PREPARATION**

(30) Priority: 30.07.2009 JP 2009177490
(71) Applicant: Idemitsu Kosan Co., Ltd., Chiyoda-ku Tokyo 100-8321 (JP)
(72) Inventor: MOCHIZUKI, Masami, Sodegaura-shi Chiba 299-0293 (JP); NAGASHIMA, Kyo, Sodegaura-shi Chiba 299-0293 (JP)
(74) Representative: Vossius & Partner
(86) International application number: PCT/JP2010/062454
(87) International publication number: WO 2011/013592

(57) **Abstract**

An object of the present invention is to formulate cashew nut shell liquid into a coated formulation which is easy to handle. Another object of the present invention is to prevent irritations caused by the cashew nut shell liquid. Still another object of the present invention is to provide a preparation which is suitable for release of the cashew nut shell liquid in the rumen. To solve the problems, provided is a coated formulation, which is produced by adsorbing cashew nut shell liquid, anacardic acid, or cardanol to an oil adsorbent, granulating the resultant product, and by coating the particle surfaces with a hardened oil or a coating agent.

## Description

### [Technical Field]

The present invention relates to a coated formulation, which is produced by adsorbing cashew nut shell liquid (CNSL) to an oil adsorbent, granulating the resultant product, and by coating the particle surfaces. The present invention also relates to a feed additive and a feed, each of which contains the coated formulation, and a method of raising a domestic animal or the like using the additive and the feed.

### [Background Art]

Cashew nut shell liquid is known to have antibacterial effect (Non-patent Document 1) and coccidiosis-relieving effect (Patent Documents 1 to 3). Moreover, as for the effect of improving the rumen function of a ruminant, the results of an in vitro test using anacardic acid (Non-patent Document 2) have been reported. In addition, cashew nut shell liquid is known to have antibacterial effect on gram-positive bacteria such as *Staphylococcus aureus, Streptococcus mutans, Bacillus subtilis,* and *Bacillus ammoniagenes,* and to have no antibacterial effect on gram-negative bacteria such as *Escherichia coli, Enterobacter aerogenes,* and *Pseudomonas aeruginosa,* and fungi such as *Saccharomyces cerevisiae, Candida utilis,* and *Penichillium chrysogenum* (Non-patent Document 3).
Cashew nut shell liquid is in a liquid or solid form at room temperature, and hence is preferably formulated to be blended uniformly in a feed. Cashew nut is a plant belonging to the family *Anacardiaceae,* and may cause irritations in a user. Therefore, a preparation containing cashew nut shell liquid which is not exposed to the surface and is released in the rumen of a ruminant is preferred. To prevent irritations, the surface of the preparation is preferably coated.
A feed coated with a hardened oil has been reported as a rumen bypass feed, but a feed which releases an active ingredient in the rumen has not been reported. For example, a vitamin E-containing feed coated with a hardened oil may bypass the rumen (Patent Document 4). Moreover, a vitamin K3-containing feed coated with a hardened oil may bypass the rumen because the feed has particle sizes of as small as 50 to 1000 µm, is not broken by rumination of a cow, and is not digested in the rumen (Patent Document 5). In addition, there has been reported a fat-coated solid feed coated with a fat mixture including an animal fat, a vegetable oil, and a hardened oil and adjusted to 40 to 50° C in its melting point (Patent Document 6). However, release in the rumen is not described.

### [Prior Art Documents]

### [Patent Documents]

[Patent Document 1] JP 2003-238400 A
[Patent Document 2] JP 2001-151675 A
[Patent Document 3] JP 08-231410 A
[Patent Document 4] JP 2005-278442 A
[Patent Document 5] JP 06-237700 A
[Patent Document 6] JP 06-217707 A

### [Non-patent Documents]

[Non-patent Document 1] Muroi, H. et al. Bioorganic & Medicinal Chemistry 12,583-587(2004)
[Non-patent Document 2] Van Nevel C. J. , et al, Applied Microbiology 21, 365-366 (1971)
[Non-patent Document 3] Himejima M. and Kubo I., J. Agric. Food Chem., 39, 418-421 (1991)

### [Summary of the Invention]

### [Problems to be solved by the Invention]

An object of the present invention is to formulate cashew nut shell liquid into a coated formulation which is easy to handle. Another object of the present invention is to prevent irritations caused by the cashew nut shell liquid. Still another object of the present invention is to provide a preparation which is suitable for release of the cashew nut shell liquid in a rumen.

### [Means for solving the Problems]

The inventors of the present invention have conducted intensive studies in order to solve the above-mentioned problems, and as a result, the inventors have found that it is possible to formulate cashew nut shell liquid into a coated formulation which is easy to handle and to prevent irritations caused by the cashew nut shell liquid, by adsorbing the cashew nut shell liquid to an oil adsorbent, granulating the resultant product, and coating the particle surfaces with a hardened oil or a coating agent.
Further, the inventors have found that the coated formulation of the present invention has excellent handling property and irritation preventing ability as well as excellent rumen function improving effect.
Thus, the inventors of the present invention have completed the present invention.

That is, the present invention is as follows:
(1) a coated formulation produced by adsorbing cashew nut shell liquid, anacardic acid, or cardanol to an oil adsorbent, granulating the resultant product, and coating the particle surfaces;
(2) a feed additive containing the coated formulation according to the item (1);
(3) a feed containing the coated formulation according to the item (1) or the feed additive according to the item (2);
(4) a method of raising livestock including feeding livestock the feed according to the item (3); and
(5) a method of manufacturing a coated formulation including adsorbing cashew nut shell liquid, anacardic acid, or cardanol to an oil adsorbent, granulating the resultant product, and coating the particle surfaces.

### [Effects of the Invention]

When the cashew nut shell liquid is processed into a coated formulation which is easy to handle and causes no irritation, a preparation which is easy to blend in a feed and is safe for a user can be obtained.
The coated formulation of the present invention can release the cashew nut shell liquid in the rumen after eating.

### [Modes for carrying out the Invention]

The coated formulation of the present invention is a formulation produced by adsorbing cashew nut shell liquid, anacardic acid, or cardanol to an oil adsorbent, granulating the resultant product, and coating the particle surfaces.

The cashew nut shell liquid is an oily liquid contained in the shell of the fruit of a cashew nut tree (*Anacardium occidentale* L.). The cashew nut shell liquid contains, as the components thereof, anacardic acid, cardanol, and cardol. In general, anacardic acid is converted into cardanol by a heating treatment.
Non-heated cashew nut shell liquid extracted by compressing the shell of a cashew nut contains 55 to 80 mass% anacardic acid, 5 to 20 mass% cardanol, and 5 to 30 mass% cardol as described in J. Agric. Food Chem. 2001, 49, 2548-2551.
Heated cashew nut shell liquid obtained by heat-treating non-heated cashew nut shell liquid at 130°C or more contains 0 to 10 mass% anacardic acid, 55 to 80 mass% cardanol, and 5 to 30 mass% cardol. This is because anacardic acid, which is a major component of non-heated cashew nut shell liquid, is converted into cardanol by decarboxylation.

The cashew nut shell liquid can be obtained as a vegetable oil extracted by compressing the shell of a cashew nut. In addition, the cashew nut shell liquid can also be obtained by extracting, e.g., solvent-extracting a cashew nut shell. Further, the cashew nut shell liquid can be obtained according to a method described in JP 08-231410 A, e.g., by a solvent extraction method.
A commercially available cashew nut shell liquid product may also be used.
The cashew nut shell liquid of the present invention may be heated cashew nut shell liquid obtained by heating non-heated cashew nut shell liquid, which was obtained by the above-mentioned method, at 70°C or more, preferably 130°C or more.
The cashew nut shell liquid of the present invention may be cashew nut shell liquid obtained by performing compression and extraction of the shell of a cashew nut and heat-treating the resultant product (non-heated cashew nut shell liquid) at 130°C.
The coated formulation of the present invention may contain anacardic acid or cardanol instead of the cashew nut shell liquid.

The content of the cashew nut shell liquid in the coated formulation of the present invention is 15 mass% to 70 mass%, preferably 20 mass% to 60 mass%, and still more preferably 25 mass% to 55 mass% based on the total amount of the coated formulation. It is preferred that the content be 15 mass% or more, because the rumen fermentation improving effect can be exhibited efficiently, and that the content be 70 mass% or less, because the handling property of the coated formulation can be maintained by eliminating hand irritations while at work.

The content of the cashew nut shell liquid in the additive for feed of the present invention is preferably 0.02 mass% to 90 mass%, more preferably 0.04 mass% to 80 mass%, and still more preferably 0.06 mass% to 70 mass%. It is preferred that the content be 0.02 mass% or more, because the rumen fermentation improving effect can be exhibited efficiently, and that the content be 90 mass% or less, because the handling property can be maintained.

The content of the cashew nut shell liquid in the feed of the present invention is preferably 0.02 mass% to 4.0 mass%, more preferably 0.04 mass% to 2.0 mass%, and still more preferably 0.06 mass% to 1.0 mass%. It is preferred that the content be 0.02 mass% or more, because the rumen fermentation improving effect can be exhibited efficiently, and that the content be 4.0 mass% or less, because the handling property can be maintained.

As anacardic acids used in the present invention, there are exemplified natural anacardic acid, synthetic anacardic acid, and derivatives thereof. Further, commercially-available anacardic acidmay be used. As described in JP 08-231410 A, anacardic acids may be obtained by eluting the cashew nut shell liquid, which has been obtained by extracting the cashew nut shell with an organic solvent, through chromatography on a silica gel column using a solvent of n-hexane, ethyl acetate, and acetic acid mixed at varied ratios (JP 03-240721 A, JP 03-240716 A, and the like), for example. Those anacardic acids may be contained in a coated formulation, an additive for feed, or a feed in the same content as that of the cashew nut shell liquid.

Examples of the cardanol used in the present invention include natural cardanol, synthetic cardanol, and derivatives thereof. In addition, the cardanol used in the present invention can be obtained by decarboxylation of anacardic acid which is a major component of cashew nut shell liquid. Those cardanols may be contained in a coated formulation, an additive for feed, or a feed in the same content as that of the cashew nut shell liquid.
In the case where heated cashew nut shell liquid is used, the mass ratio of anacardic acid and cardanol in the heated cashew nut shell liquid is preferably 0:100 to 20:80.

As the oil adsorbent used in the present invention, there are exemplified bentonite, zeolite, attapulgite, sepiolite, perlite, diatomite, silica, activated carbon, activated earth, and acid earth, and granular products are preferred. The oil adsorbent of the present invention preferably adsorbs an oil in an amount of 50 to 300 g per 100 g of the adsorbent. In addition, the particle size of the adsorbent is preferably 2 to 200 µm because the particles become coarse to cause separation when the particle size exceeds 200 µm.

The coated formulation of the present invention can be obtained as follows, for example.
The oil adsorbent and cashew nut shell liquid are mixed, and the mixture is granulated using a usual extrusion granulator, if required, followed by tableting.
Meanwhile, the mixture of the oil adsorbent and cashew nut shell liquid can be compressed to tablet by using a briquette machine.
Note that, a feed ingredient such as farina can be mixed with the oil adsorbent and cashew nut shell liquid, and granulated together.
After granulation, a rounding treatment may be performed to round off corners of the pellets for chamfering. The rounding treatment may be performed using, for example, a marumerizer.

In the present invention, the coated formulation of the present invention may be produced by performing: granulation; if required, a rounding treatment; and a coating treatment with, for example, a hardened oil or a coating agent.
Examples of the hardened oil used in the present invention include extremely hardened oils such as extremely hardened soybean oil, extremely hardened palm oil, and extremely hardened rapeseed oil, but the oil is not limited thereto so long as cashew nut shell liquid, anacardic acid, or cardanol can be released in the rumen.
Examples of the coating agent used in the present invention include zein (manufactured by Kobayashi Perfumery Co., Ltd.), shellac (manufactured by Gifu Shellac manufacturing Co., Ltd.), HPMC (hydroxypropyl methylcellulose: product name Metolose, manufactured by Shin-Etsu Chemical Co., Ltd.), pullulan (manufactured by Hayashibara Shoji, Inc.), and hemilose (manufactured by Freund), a sugar such as sucrose, lactose, and torehalose, a water-soluble sheet such as an oblate and a water-soluble paper but the agent is not limited thereto so long as cashew nut shell liquid, anacardic acid, or cardanol can be released in the rumen.
The granule or tablet also can be coated by a powdery coating agent. Such coating agent includes farina such as starch and flour, and a material used for the above mentioned oil adsorbent.
In the case where the coating treatment is performed using the hardened oil, the following steps are preferably performed after granulation. Particles are added to a hardened oil dissolved, and only the particles are separated and cooled. The coating treatment is repeated to adjust the thickness.
In the case where the coating treatment is performed using the coating agent, the following steps are preferably performed after granulation. An aqueous solution of the coating agent or a solution of the coated formulation in ethanol is sprayed to particles, and the particles are dried. The coating treatment is repeated to adjust the thickness.

The mass ratio of the particles and the hardened oil or coating agent in the coated formulation of the present invention is preferably 85 to 99.9:15 to 0.1.

The coating may be a multilayer obtained by overcoating two or more kinds of hardened oils and/or coating agents. Before the coating treatment using the hardened oil or coating agent, an oil adsorbent such as bentonite, zeolite, attapulgite, sepiolite, perlite, diatomite, silica, activated carbon, activated earth, or acid earth is preferably applied to prevent the sticky property of the mixture of the cashew nut shell liquid and adsorbent.
The coating agent is preferably zein, shellac, HPMC (hydroxypropyl methylcellulose), pullulan, and hemilose.
In addition, application of the oil adsorbent and application of the hardened oil or coating agent may be repeated twice or three times or more, respectively.

The coated formulation of the present invention is preferably used as a rumen fermentation improving agent for ruminants, a bloat controlling agent, a bloat therapeutic agent, an acidosis therapeutic agent, an agent for controlling a disease caused by *Clostridium,* an agent for controlling a disease caused by *Coccidia,* or a body weight gain agent for a domestic animal.

The additive for feed of the present invention is not particularly limited so long as the additive for feed contains the coated formulation of the present invention, but the additive for feed may further contain an arbitrary component such as a component which is effective for the growth promotion of a ruminant, a nutritional supplement component, or a component for enhancing the preservation stability. Examples of the arbitrary components include: probiotics such as *Enterococcus, Bacillus,* and *Bifidus;* enzymes such as amylase and lipase; vitamins such as L-ascorbic acid, choline chloride, inositol, and folic acid; minerals such as potassium chloride, iron citrate, magnesium oxide, and phosphoric salts; amino acids such as DL-alanine, DL-methionine, and L-lysine; organic acids such as fumaric acid, butyric acid, lactic acid, and acetic acid, and salts thereof; antioxidants such as ethoxyquin, dibutylhydroxytoluene, butylhydroxyanisol, ferulic acid, vitamin C, and vitamin E; a fungicide such as calcium propionate; binders such as CMC, casein sodium, and sodium polyacrylate; emulsifiers such as lecithin, glycerin fatty acid ester, and sorbitan fatty acid ester; pigments such as astaxanthin and canthaxanthin; and flavoring agents such as various esters, ethers, and ketones.

In addition, the additive for feed of the present invention may be used as a feed by mixing the additive for feed with another feed component used in a usual feed. The kind of the feed and the components other than the coated formulation are not particularly limited. The feed is preferably a feed for ruminants.

The feed of the present invention is suitable for raising ruminants such as cows, goats, and sheep. The amount of feed to be ingested by an animal may be appropriately adjusted depending on the species of animals, body weight, age, sex, health condition, feed component, etc. The amount of cashew nut shell liquid contained in the feed is preferably 0.005 to 500 g per animal per day, more preferably 0.05 to 100 g per animal per day, and still more preferably 0.5 to 50 g per animal per day.
Any method normally used may be adopted as a method of feeding animals and a method of raising animals depending on the species of animals.

### [Examples]

500 kg of cashew nut shells were purchased from Cashew Trading Co. , Ltd. , and the shells were compressed, thereby producing 158 kg of cashew nut shell liquid (non-heated CNSL). Meanwhile, heat-treated cashew nut shell liquid (heated CNSL) undergone a heat-treatment to convert anacardic acid into cardanol was also purchased from Cashew Trading Co., Ltd.
The composition of CNSL was measured by the following method. That is, HPLC (Waters 600, Nihon Waters K.K.), a detector (Waters 490E, Nihon Waters K.K.), a printer (Chromatopak C-R6A, Shimadzu Corporation), and a column (SUPELCOSIL LC18, SUPELCO, Inc.) were used. A solvent including acetonitrile:water:acetic acid=80:20:1 (volume ratio) was used, and the flow rate was adjusted to 2 ml/min. Detection was performed at an absorbance of 280 nm.
The non-heated cashew nut shell liquid was found to contain 61.8 mass% anacardic acid, 8.2 mass% cardanol, and 19.9 mass% cardol, while the heated cashew nut shell liquid was found to contain 0.0 mass% anacardic acid, 71.4 mass% cardanol, and 14.4 mass% cardol.

### 1. Comparative Example

600 g of silica (Sipernat 22, manufactured by Evonik Degussa Japan Co. , Ltd. ) and 1200 g of non-heated CNSL were kneaded, followed by extrusion granulation (Φ3.0 mm). A marumerizer Q400 (manufactured by Techno Pad Dalton, KK) was used for a rounding treatment. The rounding treatment was performed at 510 rpm for 60 seconds. The particles were easy to adhere, resulting in increasing the particle size (Preparation G, Comparative Example 1).
1170 g of silica (Sipernat 22, manufactured by Evonik Degussa Japan Co. , Ltd.) and 2340 g of non-heated CNSL were kneaded, followed by extrusion granulation (Φ3.0 mm). The marumerizer Q400 (manufactured by Techno Pad Dalton, KK) was used for a rounding treatment. After the rounding treatment at 510 rpm for 30 seconds, 95 g of bentonite (Na-bentonite, manufactured by Kunimine Industries Co., Ltd.) was added, and 95 g of bentonite was added 30 seconds later. The rounding treatment is further continued for 30 seconds, thereby producing Preparation H (Comparative Example 2) of Φ3 to 4 mm the surface of which was coated with bentonite.

### 2. Example

2 g of shellac (manufactured by Gifu Shellac manufacturing Co., Ltd.) was dissolved in 100 ml of ethanol. The solution was sprayed to 100 g of Preparation H (Comparative Example 2) using a hand spray to adhere shellac uniformly on the surface. The adhesion amount was 6.0 g. Drying was performed at 50°C for 1 hour. The above-mentioned procedure was repeated four times, to thereby obtain Coated formulation A (Example 1) coated with 0.5 mass% shellac.
2 g of zein (manufactured by KOBAYASHI PERFUMERY Co., Ltd.) was dissolved in 100 ml of 70 mass% ethanol aqueous solution. The solution was sprayed to 100 g of Preparation H (Comparative Example 2) using a hand spray to adhere zein uniformly on the surface. The adhesion amount was 5.3 g. Drying was performed at 50°C for 1 hour. The above-mentioned procedure was repeated four times, to thereby obtain Coated formulation B (Example 2) coated with 0.5 mass% zein.
2 g of HPMC (hydroxypropyl methylcellulose: product name Metolose, manufactured by Shin-Etsu Chemical Co., Ltd.) was dissolved in 100 ml of pure water. The solution was sprayed to Preparation H (Comparative Example 2) using a hand spray to adhere HPMC uniformly on the surface. The adhesion amount was 10 g. Drying was performed at 50°C for 1 hour. The above-mentioned procedure was repeated twice, to thereby obtain Coated formulation C (Example 3) coated with 0.6 mass% HPMC.
2 g of pullulan (manufactured by HAYASHIBARA SHOJI, INC.) was dissolved in 100 ml of pure water. The solution was sprayed to Preparation H (Comparative Example 2) using a hand spray to adhere pullulan uniformly on the surface. The adhesion amount was 8.6 g. Drying was performed at 50°C for 1 hour. The above-mentioned procedure was repeated twice, to thereby obtain Coated formulation D (Example 4) coated with 0.6 mass% pullulan.
9 g of hemilose (manufactured by Freund) was dissolved in 10 ml of ethanol. The solution of hemilose in ethanol was dissolved in 79.2 ml of pure water. Moreover, 0.9 g of D-sorbitol and 0.9 g of sucrose fatty acid ester (S-1170, manufactured by Mitsubishi-Kagaku Foods Corporation) were dissolved. The solution was sprayed to 100 g of Preparation H (Comparative Example 2) using a hand spray to adhere the coating solution uniformly on the surface. The adhesion amount was 5.0 g. Drying was performed at 50°C for 1 hour. The above-mentioned procedure was repeated, to thereby obtain Coated formulation E (Example 5) coated with 0.9 mass% hemilose.
1. 0 g of extremely hardened palm oil (manufactured by Yokozeki Oil & Fat Industries Co. , Ltd. ) was placed in a 200-ml plastic beaker, and dissolved by heating to 60° C. 50 g of Preparation H (Comparative Example 2) was added and mixed to adhere the hardened oil uniformly on the surface. The whole was cooled to room temperature, to thereby obtain a preparation coated with the hardened oil. The above-mentioned procedure was repeated ten times, to thereby obtain Coated formulation F (Example 6) coated with the 13 mass% hardened oil.
The physical properties of the preparations of Comparative Examples and the coated formulation of Examples are shown in Table 1.

### Physical properties of preparations and coated formulations

| | | |
|---|---|---|
| Completely dispersible and no sticky feeling | | 0 |
| Extremely slight sticky feeling | | 1 |
| Sticky feeling | | 2 |
| Preparations adhere to each other to form balls | | 3 |
| Clayey | | 4 |
| | Highest score | 4 |

**[Table 1]**

| | Physical properties of preparations and coated formulations |
|---|---|
| Preparation G (Comparative Example 1) | 3 |
| Preparation H (Comparative Example 2) | 2 |
| Coated formulation A (Example 1) | 1 |
| Coated formulation B (Example 2) | 1 |
| Coated formulation C (Example 3) | 1 |
| Coated formulation D (Example 4) | 1 |
| Coated formulation E (Example 5) | 1 |
| Coated formulation F (Example 6) | 0 |

The effects of the preparations and coated formulations on irritations were examined for an adult. As a control, 50 µL of non-heated CNSL was placed on the left arm of a man in his forties and wiped off 30 seconds later. Three drops of each of the above-mentioned Examples 1 to 6 were placed on the left arm of the man in his forties and wiped off 30 seconds later. In the same way as above, three drops of each of the above-mentioned preparations of Comparative Examples 1 and 2 and coated formulations were placed on the left arm of the man in his forties and wiped off 30 seconds later. Observation was performed over one week every day, and skin reactions were evaluated based on the following criteria.

### Formation of erythema and crust

| | | |
|---|---|---|
| No erythema | | 0 |
| Extremely slight erythema (barely detectable) | | 1 |
| Obvious erythema | | 2 |
| Middle or high degree of erythema | | 3 |
| From high degree of erythema to form of slight crust (deep damage) | | 4 |
| | Highest score | 4 |

**[Table 2]**

| | Day 1 | Day 2 | Day 3 | Day 4 | Day 5 | Day 6 | Day 7 |
|---|---|---|---|---|---|---|---|
| Non-heated CNSL | 0 | 1 | 4 | 4 | 4 | 4 | 4 |
| Preparation G (Comparative Example 1) | 0 | 1 | 2 | 2 | 1 | 1 | 0 |
| Preparation H (Comparative Example 2) | 0 | 1 | 2 | 2 | 1 | 1 | 0 |
| Coated formulation A (Example 1) | 0 | 0 | 0 | 0 | 0 | 0 | 0 |
| Coated formulation B (Example 2) | 0 | 0 | 0 | 0 | 0 | 0 | 0 |
| Coated formulation C (Example 3) | 0 | 0 | 0 | 0 | 0 | 0 | 0 |
| Coated formulation D (Example 4) | 0 | 0 | 0 | 0 | 0 | 0 | 0 |
| Coated formulation E (Example 5) | 0 | 0 | 0 | 0 | 0 | 0 | 0 |
| Coated formulation F (Example 6) | 0 | 0 | 0 | 0 | 0 | 0 | 0 |

Coating with the coating agent or hardened oil was found to prevent irritations.

### 3. Rumen function improvement example

### (1) Culture

As a culture inoculum, a rumen liquor collected orally from a beef cattle using a rumen cannula (double gauze filtrate) was used. The inoculum was diluted two-fold with McDougal's artificial saliva (pH 6.8) before use.
The composition of the McDougal's artificial saliva (pH 6.8) is shown below.

| | |
|---|---|
| Distilled water | 1 L |
| MgCl₂·6H₂O | 0.13 g |
| Na₂HPO₄ | 3.68 g |
| CaCl₂·2H₂O | 0.053 g |
| KCl | 0.57 g |
| NaCl | 0.47 g |
| NaHCO₃ | 9.80 g/pH 6.8 |

The concentration of the non-heated CNSL in a test culture was adjusted to 500 mg/L. 0.05 g of CNSL was dissolved in 1 ml of ethanol, and 100 µl of the solution was added to a screw-top test tube. The test tube was left standing for several hours to volatile ethanol. To the test tube were added 0.15 g of corn starch, 0.025 g of formula feed powder, and 0.025 g of dried orchardgrass powder as culture substrates. 10 ml of the diluted rumen liquor was added, and a butyl rubber cap and a plastic screw cap were put while blowing nitrogen gas into the head space, and anaerobic culture was performed with shaking at 70 rpm (37°C, 18 hours).
A preparation mixed in a feed is considered to be broken by chewing of a domestic animal. To model the phenomenon, the preparations were pounded in a mortar. Preparation H (Comparative Example 2) was pounded in a mortar and added to a screw-top test tube in an amount of 0.0079 g so that the final concentration of the non-heated CNSL in the test culture might be 500 mg/L, and a test was performed in the same way as above. Coated formulation F in Example 6 was pounded in a mortar and added to a screw-top test tube in an amount of 0.0089 g, and a test was performed in the same way as above.
Treatments include no addition (only ethanol: control group), non-heated CNSL group, preparation group, and coated formulation group, and culture was performed in quintuplicate.

### (2) Analysis

Methane was analyzed by FID gas chromatography. Specifically, there were used gas chromatography (GC-9A, Shimadzu Corporation), Chromatopak (C-R4A, Shimadzu Corporation), column (Molecular Sieve 5A; 60-80 mesh; 2.5 mx3.0 mm; stainless column, Shinwa Chemical Industries Ltd.), and N₂ carrier gas. The total volatile fatty acid (VFA) concentration and composition were analyzed by FID gas chromatography. Specifically, measurement was performed in the same way as in the case of methane except that there was used a column (Reoplex 400; 10%; ChromosorbW; 80-100 mesh; AW-DMCS; 2.1 m×3.2 mm; glass column, Shinwa Chemical Industries Ltd.).

### (3) Results

### (i) Gas formation (Table 3)

Methane was found to decrease by addition of the coated formulation of the present invention, and the decrease degree was almost the same as that of the non-heated CNSL.

**[Table 3]**

| Amount of methane generated | |
|---|---|
| | Amount of methane generated (ml) |
| Non-treated | 2.07±0.01 |
| Non-heated CNSL | 0.07±0.01 |
| Preparation H (Comparative Example 2) | 0.08±0.01 |
| Coated formulation F (Example 6) | 0.10±0.02 |

### (ii) Formation of volatile fatty acid (VFA) (Table 4)

The concentration of total volatile fatty acid was not changed by addition of the coated formulation (the coated formulation did not suppress fermentation itself). However, the fermentation pattern was markedly changed to significantly decrease the generation of acetic acid and butyric acid and to significantly increase the generation of propionic acid. The increase degree was almost the same as that of the non-heated CNSL.

**[Table 4]**

| Amount of VFA generated (g/L) | | | | |
|---|---|---|---|---|
| | Total VFA | Acetic acid | Propionic acid | Butyric acid |
| Non-treated | 10.29±0.21 | 5.62±0.11 | 3.24±0.05 | 1.14±0.04 |
| Non-heated CNSL | 10.43±0.21 | 4.41±0.12 | 5.61±0.10 | 0.31±0.01 |
| Preparation H (Comparative Example 2) | 10.67±0.30 | 4.62±0.10 | 5.63±0.20 | 0.33±0.01 |
| Coated formulation F (Example 6) | 10.68±0.10 | 4.58±0.06 | 5.68±0.06 | 0.33±0.01 |

As is clear from Table 3 and Table 4, the rumen function improving effect of the coated formulation of the present invention is almost the same as that of the non-heated CNSL, which reveals that the non-heated CNSL was released from the coated formulation into the culture.

### [Industrial Applicability]

When the coated formulation of the present invention is processed into granules, the handling property can be improved by solving the problem of difficulty in mixing in feeds, caused by CNSL which is a greasy liquid and sticky. In addition, when a CNSL stock solution adheres to the skin, the solution cannot be removed, resulting in causing irritations. However, it is possible not to contact the oil with the skin and to prevent the irritations by adsorbing the solution in an oil adsorbent and coating the particle surfaces.
The coated formulation of the present invention has rumen fermentation improving effect, bloat controlling effect, bloat therapeutic effect, acidosis therapeutic effect, effect for controlling a disease caused by *Clostridium,* an effect for controlling a disease caused by *Coccidia,* or body weight gain effect for a domestic animal.

## Claims

1. A coated formulation, which is produced by:
adsorbing cashew nut shell liquid, anacardic acid, or cardanol to an oil adsorbent;
granulating a resultant product; and
coating particle surfaces with a hardened oil or a coating agent.

2. An additive for feed, comprising the coated formulation according to claim 1.

3. A feed comprising the coated formulation according to claim 1 or the additive for feed according to claim 2.

4. A method of raising a domestic animal, comprising causing the animal to ingest the feed according to claim 3.

5. A method of manufacturing a coated formulation comprising:
adsorbing cashew nut shell liquid, anacardic acid, or cardanol to an oil adsorbent;
granulating the resultant product; and
coating the particle surfaces.
